# EUROPEAN PATENT APPLICATION

(11) **EP 1 529 535 A1**
(43) Date of publication of application: **11.05.2005**
(21) Application number: 04026263.6
(22) Date of filing: 05.11.2004
(51) Int. Cl.: A61K 38/34, A61K 38/33, C07K 14/69, C07K 14/665

(54) **Isolation of a circulating human lipolytic peptide**

(30) Priority: 06.11.2003 DE 10352335; 22.04.2004 EP 04009526
(71) Applicant: IPF PharmaCeuticals GmbH, 30625 Hannover (DE)
(72) Inventor: Maronde, Erik, Dr., 30900 Wedemark (DE); Fricke, Katrin, 30625 Hannover (DE)
(74) Representative: Meyers, Hans-Wilhelm, Dr.Dipl.-Chem.

(57) **Abstract**

The present invention discloses a polypeptide obtainable from human hemofiltrate having a least 90% homology to a polypeptide having the amino acid sequence Z¹x¹x²x³x⁴x⁵x⁶x⁷x⁸x⁹px¹⁰ x¹¹mehfrwgx¹²ppx¹³x¹⁴Z² along with a process to obtain said polypeptide. The use of the polypeptide according to the invention for manufacturing of a medicament for the treatment of adipositas or obesity or a disease related with adipositas or obesity is also disclosed. Furthermore a nucleic acid coding for the polypeptide according to the invention together with a vector comprising said nucleic acid is disclosed.

SEQUENCE LISTING

<110> IPF PharmaCeuticals GmbH

<120> Isolation of a circulating human lipolytic peptide
      042564
<130> 032305ep/Me-FM

<140>
<141>

<160> 3

<170> PatentIn Ver. 2.1

<210> 1
<211> 24
<212> PRT
<213> Homo sapiens

<400> 1
Val Ala Ala Glu Lys Lys Asp Glu Gly Pro Tyr Arg Met Glu His Phe
  1               5                  10                  15

Arg Trp Gly Ser Pro Pro Lys Asp
             20


<210> 2
<211> 22
<212> PRT
<213> Homo sapiens

<400> 2
Ala Glu Lys Lys Asp Glu Gly Pro Tyr Arg Met Glu His Phe Arg Trp
  1               5                  10                  15

Gly Ser Pro Pro Lys Asp
             20


<210> 3
<211> 75
<212> DNA
<213> Homo sapiens

<400> 3
gtggcggccg agaagaagga cgagggcccc tacaggatgg agcacttccg ctggggcagc 60
ccgcccaagg acaag                                                  75

## Description

### Background of the invention

In the developed and developing countries the incidence of adipositas or obesity has grown epidemically. The reasons for this epidemic increase are not known but surely are related to overeating and lack of exercise, but also genetic and viral influences are discussed.

Some metabolic diseases partly due to mutations in genes related to eating behaviour, energy expenditure and energy use have been described. It is more or less agreed on that every slight super-optimal food consumption in combination with lack of exercise which is for many people in the modern western civilisation part of their all day life often represents the starting point of a fatal progressive weight gain.

No matter what the reason for the obesity was in the beginning, in the end obese people possess a rather large portion of either subcutaneous or visceral or both white fats, a tissue composed of mainly so-called white adipocytes. These cells store unused energy in the form of triglycerides in their fat vesicles. The release of these triglycerides is in normal weight people meant for the supply of extra energy when the glycogen and protein stores are exhausted. This usually does not happen to people living the western life style but occurs for example in workers, long distance runners or triathlets. The release of the triglycerides to the circulation is regulated by hormonal systems, the longest known and most important of which is the beta-adrenergic system which consists of epinephrine or norepinephrine which is released from nerve endings reaching into the white adipose tissue and a beta-adrenergic receptor on the adipocytes. When due to the general physiological conditions triglycerides are needed norepinephrine is released from these nerve endings, reaches the white adipocyte and binds to the beta-adrenergic receptor residing on the surface of the adipocyte. This binding of norepinephrine leads to a conformational change of the receptor that enables it to couple to a so-called G-protein which then couples to an enzyme called adenylate cyclase. This enzyme converts ATP to cAMP which binds to and activates a protein kinase called cAMP-activated protein kinase (or PKA) which dissociates and releases an intracellular catalytic activity that phosphorylates intracellular proteins. Among these proteins is the enzyme "hormone-sensitive-lipase" or HSL whose activity raises after phosphorylation by PKA. Also other PKA-phosphorylated proteins like perilipin change their behaviour so that stored fat from the fat vesicles of the white adipocyte is converted into releaseable forms finally leading to an increased availability of energy in the form of tri- di- and monoglycerides that can be used for the generation of energy in the body e.g. for muscle movement.

The only current FDA-approved pharmacological treatment options for obesity are Sibutramin (a centrally acting serotonin reuptake inhibitor) and Xenical (an intestinal lipase inhibitor). Beside that only caloric restriction and surgical removal of fat mass are common. There is a clear unmet medical need for other treatment options.

### Summary of the invention

A dysregulation of the fat-storing system in favor of storage rather that release of free fatty acids contributes to the condition of obesity. One object of the invention is to identify substances or hormones that lead to increased mobilisation of stored fat. Such substances could also support degradation of fat stores that are already diminished by an ongoing diet. Under diets often the first weight loss is due to water being released from the body, then glycogen and protein levels are also declining. Treatment of dieting people with supplementary substances to increase and favor the degradation of fat versus the other energy stores is therefore also desirable.

According to the invention a polypeptide solves the above addressed object. The polypeptide of the invention is obtainable from human hemofiltrate by a process comprising the steps:
- loading hemofiltrate to a cation exchange column,
- eluting with a pH gradient by buffers with increasing pH from 3.6 to 9.0
- pooling fractions and subjecting said fractions to reverse phase chromatography, and
- collecting fractions which are applied to differentiated mouse adipocytes and measuring the production of free fatty acids by an enzymatic assay estimating glycerol content in the cell supernatant.

According to the invention 3T3L1-derived differentiated mouse adipocytes can be used as appropriate cell-system. These cells are treated with fractions of human hemofiltrate and identified using a glycerol-estimation method (GPO-Trinder reagent, Sigma, Deisenhofen, Germany) lipolytic activity. This activity was found to be protease-sensitive, dose-dependent and susceptible to chromatographic separation. It was subsequently isolated in several chromatographic steps using reverse-phase and cation-exchange chromatographic material.

In a particular embodiment the polypeptide of the present invention has at least 90% homology to a polypeptide having the amino acid sequence
**Z**^{**1**}**x**^{**1**}**x**^{**2**}**x**^{**3**}**x**^{**4**}**x**^{**5**}**x**^{**6**}**x**^{**7**}**x**^{**8**}**x**^{**9**}**px**^{**10**} **x**^{**11**}**mehfrwgx**^{**12**}**ppx**^{**13**}**x**^{**14**}**Z**^{**2**}
- **x**^{**1**}: represents a hydrophobic amino acid or its derivative in particular **V** or a deletion
- **x**^{**2**}: represents a hydrophobic amino acid or its derivative in particular **A** or a deletion
- **x**^{**3**}: represents a hydrophobic amino acid or its derivative in particular **A**
- **x**^{**4**}: represents an acidic amino acid or its derivative in particular **E**
- **x**^{**5**}: represents a cationic amino acid or its derivative in particular **K**
- **x**^{**6**}: represents a cationic amino acid or its derivative in particular **K**
- **x**^{**7**}: represents an acidic amino acid or its derivative in particular **D**
- **x**^{**8**}: represents an acidic amino acid or its derivative in particular **E**
- **x**^{**9**}: represents G its derivative in particular **G**
- **x**^{**10**}: represents an aromatic amino acid or its derivative in particular **Y**
- **x**^{**11**}: represents a cationic amino acid or its derivative in particular **R**
- **M**: can be replaced by **C** or **M/C** derivatives
- **E**: can be replaced any acidic amino acid or its amino acid derivative
- **H**: can be replaced by **H** or its derivative
- **F**: can be replaced an aromatic amino acid or its derivative
- **R**: can be replaced by a cationic amino acid or its derivative
- **W**: can be replaced by an aromatic amino acid or its derivative
- **G**: can be replaced by **G** or its derivative
- **x**^{**12**}: represents a hydroxylated amino acid or its derivative in particular **S**
- **x**^{**13**}: represents a cationic amino acid or its derivative in particular **K**
- **x**^{**14**}: represents an acidic amino acid or its derivative in particular **D**
wherein each derivative of an amino acid is selected from the group consisting of a functional homologue, of alkylated, amidated, acetylated, phosphorylated esterified ether or thioether modification and/or glycosylation at the side chains of an amino acid in the polypeptide chain but essentially maintaining the biological activity of the polypeptide,
- **Z**^{**1**}: represents NH₂ or any modification of the amino terminal in particular a PEG-ylated, acetylated, alkylated derivative
- **Z**^{**2**}: represents COOH or any modification of the carboy terminal in particular an amidated, esterified derivative.

In another embodiment of the invention the polypeptide according to the invention has the following sequence:

Seq ID No: 1 vaaekkdegp yrmehfrwgs ppkd.

The polypeptide of the invention can be used as a medicament comprising an effective amount of the polypeptide of the invention and a pharmaceutically acceptable carrier. In the medicament of the invention polypeptides of the following structure can be used
**Z**^{**1**}**x**^{**1**}**x**^{**2**}**x**^{**3**}**x**^{**4**}**x**^{**5**}**x**^{**6**}**x**^{**7**}**x**^{**8**}**x**^{**9**}**px**^{**10**} **x**^{**11**}**mehfrwgx**^{**12**}**ppx**^{**13**}**x**^{**14**}**Z**^{**2**}
- **x**^{**1**}: represents a hydrophobic amino acid or its derivative in particular **V** or a deletion
- **x**^{**2**}: represents a hydrophobic amino acid or its derivative in particular **A** or a deletion
- **x**^{**3**}: represents a hydrophobic amino acid or its derivative in particular **A**
- **x**^{**4**}: represents an acidic amino acid or its derivative in particular **E**
- **x**^{**5**}: represents a cationic amino acid or its derivative in particular **K**
- **x**^{**6**}: represents a cationic amino acid or its derivative in particular **K**
- **x**^{**7**}: represents an acidic amino acid or its derivative in particular **D**
- **x**^{**8**}: represents an acidic amino acid or its derivative in particular **E**

- **x**^{**9**}: represents **G** its derivative in particular **G**
- **x**^{**10**}: represents an aromatic amino acid or its derivative in particular **Y**
- **x**^{**11**}: represents a cationic amino acid or its derivative in particular **R**
- **M**: can be replaced by **C** or **M/C** derivatives
- **E**: can be replaced any acidic amino acid or its amino acid derivative
- **H**: can be replaced by **H** or its derivative
- **F**: can be replaced an aromatic amino acid or its derivative
- **R**: can be replaced by a cationic amino acid or its derivative
- **W**: can be replaced by an aromatic amino acid or its derivative
- **G**: can be replaced by **G** or its derivative
- **x**^{**12**}: represents a hydroxylated amino acid or its derivative in particular **S**
- **x**^{**13**}: represents a cationic amino acid or its derivative in particular **K**
- **x**^{**14**}: represents an acidic amino acid or its derivative in particular **D**
wherein each derivative of an amino acid is selected from the group consisting of a functional homologue, of alkylated, amidated, acetylated, phosphorylated esterified ether or thioether modification and/or glycosylation at the side chains of an amino acid in the polypeptide chain but essentially maintaining the biological activity of the polypeptide,
- **Z**^{**1**}: represents NH₂ or any modification of the amino terminal in particular a PEG-ylated, acetylated, alkylated derivative
- **Z**^{**2**}: represents COOH or any modification of the carboy terminal in particular an amidated, esterified derivative.

In particular, the polypeptides have the following structure:
Seq ID No: 1 vaaekkdegp yrmehfrwgs ppkd or
Seq ID No: 2 aekkdegp yrmehfrwgs ppkd.

Seq. ID No 2 was identified as beta-melanocyte-stimulating hormone (β-MSH). This peptide is a product of the proopiomelanocortin gene which is the precursor molecule of several bioactive peptides and is mainly expressed in the hypothalamus of the brain. The longer peptide has to our knowledge not been described before from human and has not been described as being lipolytically active.

The polypeptides of the invention can be used for manufacturing of a medicament for the treatment of adipositas or obesity or a disease related with adipositas or obesity. Thus the polypeptides can be used in a method of treatment of such diseases.

In the course of the disease related with adipositas or obesity diseases of the circulation system, cardiac dysfunctions, diabetes, disorders of the skeletal system, food intake or erectile dysfunction may appear which can be treated as well.

Subject of the present invention is also any nucleic acid coding for the polypeptide of the invention or its precursor protein. In particular the respective nucleic acid has the sequence of Seq ID No: 3

gtg gcg gcc gag aag aag gac gag ggc ccc tac agg atg gag cac ttc cgc tgg
ggc agc ccg ccc aag gac aag

A vector comprising a nucleic acid of the invention is also subject of the invention as well as a cell transfected with the vector of the invention or transformed with a nucleic acid of the invention.

### Brief description of the drawings

- Figure 1:: Dose-dependent induction of lipolysis by the crude activity
- Figure 2:: Degradability of the crude activity by the protease subtilisin
- Figure 3:: Isolation of vaaekkdegp yrmehfrwgs ppkd (Seq ID No. 1) from human hemofiltrate using differentiated mouse adipocytes
- Figure 4:: Isolation of aekkdegp yrmehfrwgs ppkd (Seq ID No. 2) from human hemofiltrate using differentiated mouse adipocytes
- Figure 5:: Confirmation of one activity as β-MSH (Seq ID No. 2) using commercially available β-MSH from Bachem
- Figure 6:: Confirmation of one activity as VA-β-MSH (Seq ID No. 1) using chemically synthesized VA-β-MSH

### Detailed description of the invention

The differentiated form of the mouse adipocyte cell line was used in combination with a glycerol estimation method for the identification of lipolytic factors from human hemofiltrate. An activity from pool 3 fractions 12 and 13 was subjected to 10 subsequent chromatographic steps and the active component was surprisingly identified by MALDI-MS and Edman-N-terminal sequencing as a 24 amino acid peptide derived from the human pro-opiomelanocortin gene. The second activity was 22 amino acids long and identical to human-β-MSH.

This observation was confirmed by the finding that chemically synthesized β-MSH, obtained from Bachem, also induced a dose-dependent induction of lipolysis with an EC₅₀ of approximately 20 nM. Surprising was the fact that "it is generally accepted that human beta-melanocyte-stimulating hormone (β-MSH) does not normally exist in humans but was merely an artifactually generated 22-amino acid peptide corresponding to a lipotropin (LPH) fragment (residues 35-56)" [Bertagna et al., 1986]. Also the presence of a 24 amino acid fragment of POMC in human hemofiltrate having a lipolytic activity was unexpected.

The substances can be used for the treatment of different forms of obesity and pathologic states that are caused by obesity.

The present invention is further described in the following non-limiting examples.

### Examples

A polypeptide was identified by using a mouse preadipocyte cell line (3T3L1) which can be chemically induced to differentiate into mature white adipocytes in culture, the use of aliqouts of human hemofiltrate preparations and a chromatographic isolation strategy that preserved biological activity throughout ten consecutive separation steps. For the characterisation of the isolated peptide sequencing and matrix-assisted-laser-desorption-mass-spectrometric (MALDI-MS) techniques were employed. The identified peptide was commercially available and showed a dose-dependent lipolytic activity in the assay thereby confirming the identity of the isolated peptide.

### Cell culture

3T3L1 cells were obtained from ATCC. They were grown in DMEM with 10% FCS, penicillin, streptomycin, and L-glutamine for proliferation. For differentiation into the mature adipocyte phenotype, the cells were plated onto 96 well plates (Costar) and treated for three days with differentiation medium (DMEM with 10% FCS, dexamethasone, insulin, L-glutamine and 8-Br-cAMP) and subsequently for three to six days with sustainment medium (DMEM with 10% FCS and L-glutamine).

### Assay

Cells differentiated as described above were then treated for five hours with the hemofiltrate fractions diluted in serum-free medium. An aliquot of the cell supernatant was mixed with the glycerol-estimation-solution (GPO-Trinder Reagent, Sigma, Garching, Germany) and incubated for 15 minutes. Then the absorbance was measured at 540 nm in a 96 well plate ELISA reader (Dynex). Absolute glycerol concentrations were estimated from a standard curve of known glycerol amounts. The GPO Trinder Reagent is a mixture of enzymes and substrates that enables to measure glycerol in solution by a colorimetric reaction. The colored product is measured at 540 nm in a spectrophotometer. The amount of colored product is directly proportional to the amount of gycerol in a solution.

### Characterisation of the activity

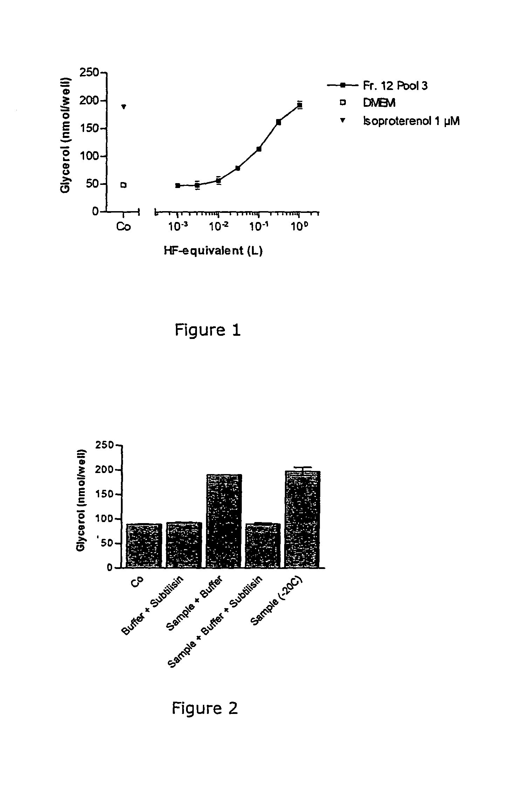

The activity from crude fraction 12 of human hemofiltrate pool 3 showed a dose-dependent effect in terms of lipolytic activity. This finding suggested that the substance acts via a receptor which is a hint for a hormonal activity. Figure 1 shows the dose-response curve of lipolytic activity from crude fraction 12 of human hemofiltrate pool 3. The experiment was performed in duplicate, and the result shown is representative for two separate experiments.

Figure 2 shows the protease-sensitivity of lipolytic activity. The peptidic character of the activity was analyzed after the third purification step. After treatment of the lipolytically active sample with Subtilisin for 4 h at 37°C, following heat deactivation of the enzyme for 10 min at 70°C, the lipolytic activity completely disappeared. This finding strongly suggests that the active component of the activity is a peptide.

### Isolation of vaaekkdegp yrmehfrwgs ppkd from human hemofiltrate

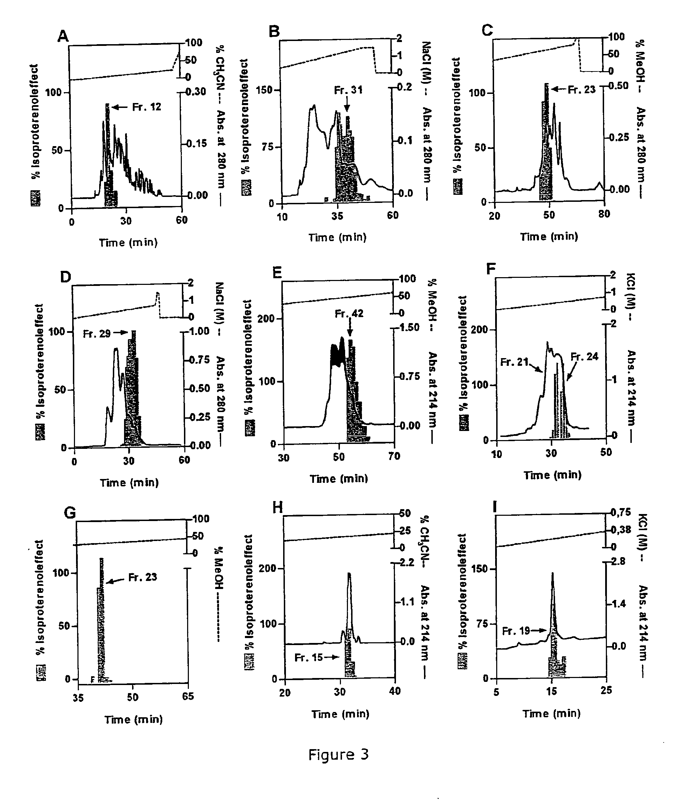

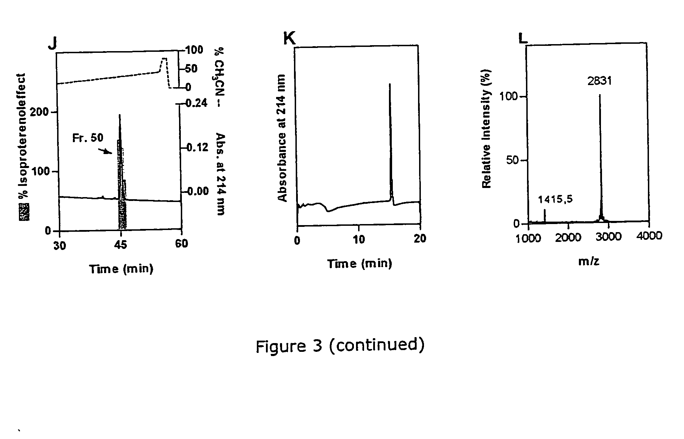

Figure 3 shows steps of the isolation of vaaekkdegp yrmehfrwgs ppkd from hemofiltrate.

Elution profiles of a preparative Fineline RPC Polymer column (3A), a TSK SP 650S cationexchange column (3B), a Bakerbond Prep Pak C₁₈ column (3C), a Biotek PepKat cationexchange HPLC column (20 x 125 mm) (3D), a Pharmacia Source C₁₅ HPLC column (3E), a Biotek PepKat cationexchange HPLC column (10 x 125 mm) (3F), a Vydac Rp-HPLC column (10 x 250 mm) (3G), a YMC ODS AQ HPLC column (10 x 250 mm) (3H), a Biotek PepKat cationexchange HPLC column (4 x 125 mm) (3I), and a Vydac Rp HPLC column (4.6 x 250 mm) (3J). Aliquots of each collected fraction were subjected to lipolysis assay. After each cation exchange chromatography step, salt was removed from each aliquot using a 96-well high performance extractions disk plate C₁₈SD (3M, St. Paul, USA) and 80% acetonitrile in 0.1% TFA as eluent.

Grey bars indicate lipolytic activity of the collected fractions. The gradient profiles are indicated by the dotted lines. Figure 3K shows the electropherogram of the purified peptide, and fig. 3L shows its MALDI-MS with the single-charged ion at m/z 2831 and the double-charged ion at m/z 1415.5.

The Fineline HPLC fractions 12 and 13 of pH-pool 3 were diluted with 10 mM HCl and loaded onto a Fineline RPC Polymer (10 x 15.5 cm) column (Amersham, Braunschweig, Germany). Elution was performed by a linear gradient increase of 80% acetonitrile from 0-30% for 53 min at a flow rate of 150 ml/min (3A). The active fraction (12) was diluted with 50 mM Na₂PO₄ (pH 4.5), loaded onto a TSK SP 650S column (Merck, Darmstadt, Germany), and peptides were eluted with a linear gradient from 0.15 to 1.5 M NaCl in 45 min at a flow rate of 50 ml/min (3B). Fractions containing the active material (26-34) were diluted with 10 mM HCl and further purified using a 47 x 300 mm Bakerbond Prep Pak C₁₈ cartridge (Waters, Milford, USA) with a gradient elution of a linear increase of MeOH from 20-80% in 63 min at a flow rate of 30 ml/min (3C). Fractions 22 and 23 were lipolytically active, diluted with 50 mM Na₂PO₄ (pH 4.5), and loaded onto a PepKat cationexchange column (20 x 150 mm) (Biotek, Östringen, Germany). A linear gradient with an increase of 1.5 M NaCl in Na₂PO₄ (pH 4.5) from 5-50% within 45 min at a flow rate of 6 ml/min was used. Active material eluted in fractions 25-30 (3D) and was subjected to the next purification step using a Source Rp C₁₅ (20 x 250 mm) column (Pharmacia, Freiburg, Germany). A linear gradient with an increase of 80% MeOH in the eluent from 20-80% in 70 min at a flow rate of 4 ml/min was performed (3E). The active of fractions 41-44 was diluted with 50 mM KH₂PO₄ (pH 4.5) and loaded onto a PepKat (10 x 125 mm) column (Biotek, Östringen, Germany). Elution was performed by a linear gradient increase of KCI concentration from 0 to 1.05 M in 70 min at a flow rate of 1.8 ml/min (3F). Active material found in fractions 21 and 22 was diluted with 10 mM HCl and loaded onto a Protein and Peptides C₁₈ (10 x 250 mm) column (Vydac, Hesperia, California, USA). Peptides were eluted with a linear gradient increase of 90% MeOH from 20-60% in 80 min at a flow rate of 1.8 ml/min (3G; no chromatogram existing). For the next purification step of the active fractions 21-23, a YMC ODS AQ (10 x 250 mm) column (YMC Europe, Scherbeck, Germany) with a gradient from 10-40% of 80% acetonitrile in 0.1% TFA in 60 min at a flow rate of 1.2 ml/min (3H) was used. Active material found in fractions 15 and 16 was diluted with 50 mM KH₂PO₄ (pH 4.5), loaded onto a PepKat (4 x 125 mm) column (Biotek, Östringen, Germany), and eluted using a linear gradient from 0 to 0.45 M KCI in 30 min at a flow rate of 1 ml/min (3I). Fractions 19 and 20 were diluted with 0.1% TFA, injected onto a Protein and Peptides C₁₈ (4.6 x 250 mm) column (Vydac, Hesperia, California, USA), and eluted with a gradient from 10-40% of 80% acetonitrile in 60 min at a flow rate of 0.7 ml/min (3J). The active fractions (50 and 51) contained purified peptide. Purity of the peptide was determined with a P/ACE MDQ capillary electrophoresis system (Beckman), using a buffer containing 0.1 M H₃PO₄ in 0.02% HPMC at a voltage of 20 kV and a fused silica column (Fig 3K). The amino acid sequence of the purified peptide was analyzed with a protein sequencer (494, Applied Biosystems) and was found to be as follows: vaaekkdegp yrmehfrwgs ppkd. The molecular mass of the purified peptide was 2831 Da and was obtained by MALDI-MS analysis (Applied Biosystems; Fig. 3L). The MALDI-MS mass and the calculated mass of the sequence obtained match within the error marges.

### Isolation of aekkdegp yrmehfrwgs ppkd from human hemofiltrate

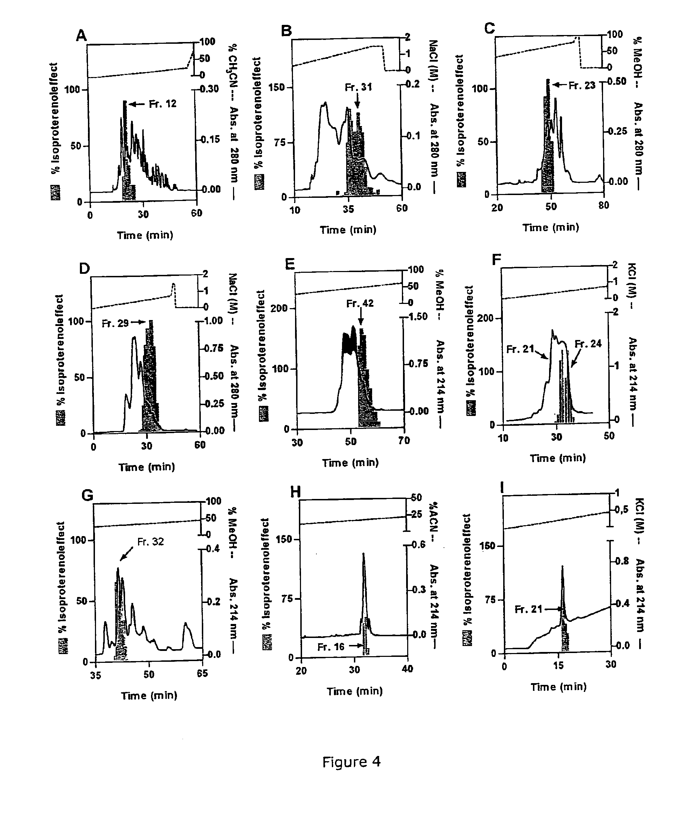

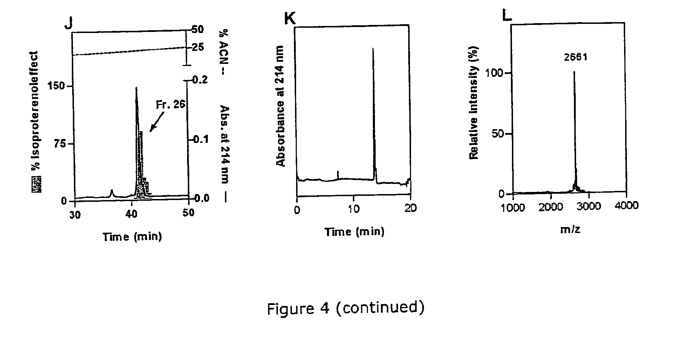

Figure 4 shows the isolation of aekkdegp yrmehfrwgs ppkd from hemofiltrate.

Elution profiles of a preparative Fineline RPC Polymer column (4A), a TSK SP 650S cation-exchange column (4B), a Bakerbond Prep Pak C₁₈ column (4C), a Biotek PepKat cation-exchange HPLC column (20 x 125 mm) (4D), a Pharmacia Source C₁₅ HPLC column (4E), a Biotek PepKat cationexchange HPLC column (10 x 125 mm) (4F), a Vydac Rp HPLC column (10 x 250 mm) (4G), a YMC ODS AQ HPLC column (4H), a Biotek PepKat cation-exchange HPLC column (4 x 125 mm) (4I), and a Vydac Rp HPLC column (4.6 250 mm) (4J). Aliquots of each collected fraction were subjected to the lipolysis assay. After each cation-exchange chromatography step, salt was removed from each aliquot using a 96-well high performance extraction disk plate C₁₈SD (3M, St. Paul, USA) with 80% acetonitrile in 0.1% TFA as eluent.

Grey bars indicate lipolytic activity of the collected fractions of one of two repetitions. The gradient profiles are indicated by the dotted lines. Figure 4 K shows the electropherogram of the purified peptide. Figure 4 L shows the MALDI-MS Analysis with a single signal at 2661 Da.

Fineline HPLC fractions 12 and 13 of pH-pool 3 were diluted with 10 mM HCl and loaded onto a Fineline RPC Polymer (10 x 15.5 cm) column (Amersham, Braunschweig, Germany). Elution was performed by a linear gradient increase of 80% acetonitrile from 0-30% for 53 min at a flow rate of 150 ml/min (Fig. 4A).

The active fraction 12 was diluted with 50 mM Na₂PO₄ (pH 4.5), loaded onto a TSK SP 650S column (Merck, Darmstadt, Germany), and peptides were eluted using a linear gradient from 0.15 to 1.5 M NaCl in 45 min at a flow rate of 50 ml/min (Fig. 4B).

Fractions containing the active material (26-34) were diluted with 10 mM HCl and further purified using a 47 x 300 mm Bakerbond Prep Pak C₁₈ cartridge (Waters, Milford, USA) with a gradient elution of a linear increase of MeOH from 20-80% in 63 min at a flow rate of 30 ml/min (Fig. 4C).

Fractions 22 and 23 were lipolytically active, diluted with 50 mM Na₂PO₄ (pH 4.5), and loaded onto a PepKat cation-exchange column (20 x 150 mm) (Biotek, Östringen, Germany). A linear gradient with an increase of 1.5 M NaCl in Na₂PO₄ (pH 4.5) from 5-50% within 45 min at a flow rate of 6 ml/min was used. Active material eluted in fractions 25-30 (Fig. 4D) and was subjected to the next purification step using a Source Rp C₁₅ (20 x 250 mm) column (Pharmacia, Freiburg, Germany). A linear gradient with an increase of 80% MeOH in the eluent from 20-80% in 70 min at a flow rate of 4 ml/min was performed (Fig. 4E).

The active fractions 41-44 were diluted with 50 mM KH₂PO₄ (pH 4.5) and loaded onto a PepKat (10 x 125 mm) column (Biotek, Östringen, Germany). Elution was performed by a linear gradient increase of KCl concentration from 0 to 1.05 M in 70 min at a flow rate of 1.8 ml/min (Fig. 4F). Active material found in fractions 24 and 25 was diluted with 10 mM HCl and loaded onto a Protein and Peptides C₁₈ (10 x 250 mm) column (Vydac, Hesperia, California, USA). Peptides were eluted with a linear gradient increase of 90% MeOH from 20-60% in 80 min at a flow rate of 1.8 ml/min (Fig. 4G).

For the next purification step of the active fractions 31-33, a YMC ODS AQ (10 x 250 mm) column (YMC Europe, Scherbeck, Germany) with a gradient from 10-40% of 80% acetonitrile in 0.1% TFA in 60 min at a flow rate of 1.2 ml/min (Fig. 4H) was used.

Active material found in fractions 16 and 17 was diluted with 50 mM KH₂PO₄ (pH 4.5), loaded onto a PepKat (4 x 125 mm) column (Biotek, Östringen, Germany), and eluted using a linear gradient from 0 to 0.45 M KCI in 30 min at a flow rate of 1 ml/min (Fig. 4I). Fractions 21-23 were diluted with 0.1% TFA, injected onto a Protein and Peptides C₁₈ (4.6 x 250 mm) column (Vydac, Hesperia, California, USA), and eluted with a gradient from 10-40% of 80% acetonitrile in 60 min at a flow rate of 0.7 ml/min (Fig. 4J).

The active fractions (25 and 26) were pooled as purified peptide.

Purity of the peptide was determined with a P/ACE MDQ capillary electrophoresis system (Beckman), using a buffer containing 0.1 M H₃PO₄ in 0.02% HPMC at a voltage of 20 kV and a fused silica column (Fig 4K). The amino acid sequence of the purified peptide was analyzed with a protein sequencer (494, Applied Biosystems) and was found to be as follows: aekkdegp yrmehfrwgs ppkd. The molecular mass of the purified peptide was 2661 Da and was obtained by MALDI-MS analysis (Applied Biosystems; Fig. 4L). The MALDI-MS mass and the calculated mass of the sequence obtained match within the error marges.

### Chemical analysis

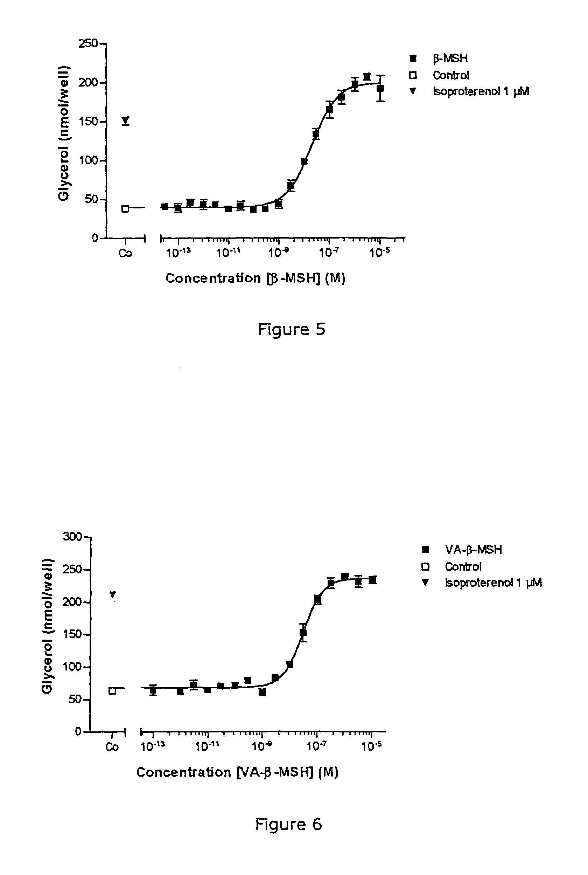

Figure 5 shows the confirmation of the finding that aekkdegpyr mehfrwgspp kd represents a lipolytic activity isolated from human hemofiltrate pool 3. Experiment was performed in triplicates, and results are representative of two independent experiments. The EC₅₀ for lipolysis is approximately 20 nM (Fig. 5).

Figure 6 shows the confirmation of the finding that vaaekkdegp yrmehfrwgs ppkd represents a lipolytic activity isolated from human hemofiltrate pool 3.

Experiment was performed in triplicates, and results are representative of two independent experiments. The EC₅₀ for lipolysis is approximately 20 nM (Fig. 6).

### Statistical Analysis

Statistical analysis was done using GraphPad Prism 3.0 (GraphPad Inc., Karlsbad, California, USA).

### References

Bertagna X, Lenne F, Comar D, Massias JF, Wajcman H, Baudin V, Luton JP, Girard F. Human beta-melanocyte-stimulating hormone revisited. Proc Natl Acad Sci U S A. 83(24): 9719-9723, 1986.

## Claims

1. Polypeptide obtainable from human hemofiltrate by a process comprising the steps:
- Loading hemofiltrate to a cation exchange column,
- Eluting with a pH gradient by buffers with increasing pH from 3.6 to 9.0
- Pooling fractions and subjecting said fractions to reverse phase chromatography, and
- Collecting fractions which are applied to differentiated mouse adipocytes and measuring the production of free fatty acids by an enzymatic assay estimating glycerol content in the cell supernatant.

2. Polypeptide having at least 90% homology to a polypeptide having the amino acid sequence
**Z**^{**1**}**x**^{**1**}**x**^{**2**}**x**^{**3**}**x**^{**4**}**x**^{**5**}**x**^{**6**}**x**^{**7**}**x**^{**8**}**x**^{**9**}**px**^{**10**} **x**^{**11**}**mehfrwgx**^{**12**}**ppx**^{**13**}**x**^{**14**}**Z**^{**2**}
**X**^{**1**} represents a hydrophobic amino acid or its derivative in particular **V** or a deletion
**x**^{**2**} represents a hydrophobic amino acid or its derivative in particular **A** or a deletion
**x**^{**3**} represents a hydrophobic amino acid or its derivative in particular **A**
**x**^{**4**} represents an acidic amino acid or its derivative in particular **E**
**x**^{**5**} represents a cationic amino acid or its derivative in particular **K**
**x**^{**6**} represents a cationic amino acid or its derivative in particular **K**
**x**^{**7**} represents an acidic amino acid or its derivative in particular **D**
**x**^{**8**} represents an acidic amino acid or its derivative in particular **E**
**x**^{**9**} represents **G** its derivative in particular **G**
**x**^{**10**} represents an aromatic amino acid or its derivative in particular **Y**
**x**^{**11**} represents a cationic amino acid or its derivative in particular **R**
**M** can be replaced by **C** or **M/C** derivatives
**E** can be replaced any acidic amino acid or its amino acid derivative
**H** can be replaced by **H** or its derivative
**F** can be replaced an aromatic amino acid or its derivative
**R** can be replaced by a cationic amino acid or its derivative
**W** can be replaced by an aromatic amino acid or its derivative
**G** can be replaced by **G** or its derivative
**x**^{**12**} represents a hydroxylated amino acid or its derivative in particular **S**
**x**^{**13**} represents a cationic amino acid or its derivative in particular **K**
**x**^{**14**} represents an acidic amino acid or its derivative in particular **D**
wherein each derivative of an amino acid is selected from the group consisting of a functional homologue, of alkylated, amidated, acetylated, phosphorylated esterified ether or thioether modification and/or glycosylation at the side chains of an amino acid in the polypeptide chain but essentially maintaining the biological activity of the polypeptide,
**Z**^{**1**} represents NH₂ or any modification of the amino terminal in particular a PEG-ylated, acetylated, alkylated derivative
**Z**^{**2**} represents COOH or any modification of the carboy terminal in particular an amidated, esterified derivative.

3. Polypeptide according to claim 1 and/or 2 having the following sequences:
Seq ID No: 1 vaaekkdegp yrmehfrwgs ppkd

4. Medicament comprising an effective amount of a polypeptide of the following structure
**Z**^{**1**}**x**^{**1**}**x**^{**2**}**x**^{**3**}**x**^{**4**}**x**^{**5**}**x**^{**6**}**x**^{**7**}**x**^{**8**}**x**^{**9**}**px**^{**10**} **x**^{**11**}**mehfrwgx**^{**12**}**ppx**^{**13**}**x**^{**14**}**Z**^{**2**}
**x**^{**1**} represents a hydrophobic amino acid or its derivative in particular **V** or a deletion
**x**^{**2**} represents a hydrophobic amino acid or its derivative in particular **A** or a deletion
**x**^{**3**} represents a hydrophobic amino acid or its derivative in particular **A**
**x**^{**4**} represents an acidic amino acid or its derivative in particular **E**
**x**^{**5**} represents a cationic amino acid or its derivative in particular **K**
**x**^{**6**} represents a cationic amino acid or its derivative in particular **K**
**x**^{**7**} represents an acidic amino acid or its derivative in particular **D**
**x**^{**8**} represents an acidic amino acid or its derivative in particular **E**
**x**^{**9**} represents **G** its derivative in particular **G**
**x**^{**10**} represents an aromatic amino acid or its derivative in particular **Y**
**x**^{**11**} represents a cationic amino acid or its derivative in particular **R**
**M** can be replaced by **C** or **M/C** derivatives
**E** can be replaced any acidic amino acid or its amino acid derivative
**H** can be replaced by H or its derivative
**F** can be replaced an aromatic amino acid or its derivative
**R** can be replaced by a cationic amino acid or its derivative
**W** can be replaced by an aromatic amino acid or its derivative
**G** can be replaced by G or its derivative
**x**^{**12**} represents a hydroxylated amino acid or its derivative in particular **S**
**x**^{**13**} represents a cationic amino acid or its derivative in particular **K**
**X**^{**14**} represents an acidic amino acid or its derivative in particular **D**
wherein each derivative of an amino acid is selected from the group consisting of a functional homologue, of alkylated, amidated, acetylated, phosphorylated esterified ether or thioether modification and/or glycosylation at the side chains of an amino acid in the polypeptide chain but essentially maintaining the biological activity of the polypeptide,
**Z**^{**1**} represents NH₂ or any modification of the amino terminal in particular a PEG-ylated, acetylated, alkylated derivative
**Z**^{**2**} represents COOH or any modification of the carboy terminal in particular an amidated, esterified derivative.

5. Use of the polypeptide of any one of the claims 1 to 3 for manufacturing of a medicament for the treatment of adipositas or obesity or a disease related with adipositas or obesity.

6. Use according to claim 5 wherein the disease related with adipositas or obesity is a disease of the circulation system, cardiac dysfunctions, diabetes, disorders of the skeletal system, food intake or erectile dysfunction.

7. A method of treating adipositas or obesity or a disease related with adipositas or obesity by administering a patient a sufficient amount of the polypeptide according to any one of the claims 1 to 3 or a medicament of claim 4.

8. A nucleic acid coding for the polypeptide of any one of the claims 1 to 3 or its precursor protein.

9. The nucleic acid of claim 8 comprising the sequence Seq ID No: 3 gcc gag aag aag gac gag ggc ccc tac agg atg gag cac ttc cgc tgg ggc agc ccg ccc aag gac aag

10. A vector comprising a nucleic acid of claim 8.

11. A cell transfected with a vector of claim 10 or transformed with a nucleic acid of claim 8.
